# EUROPEAN PATENT SPECIFICATION

(11) **EP 1 735 002 B1**
(45) Date of publication and mention of the grant of the patent: **08.07.2009**
(21) Application number: 05717999.6
(22) Date of filing: 10.03.2005
(51) Int. Cl.: A61K 45/06, A61K 31/167, A61K 31/195, A61K 31/202, A61P 35/00

(54) **CANCER THERAPY COMPRISING A NUCLEAR RECEPTOR LIGAND AND A HDAC INHIBITOR**
KREBSTHERAPIE MIT EINEM NUKLEÄREN REZEPTORLIGANDEN UND EINEM HDAC-HEMMER
THERAPIE DU CANCER AVEC DES LIGANDS DE RECEPTEURS ET DES INHIBITEURS D'HDAC

(30) Priority: 10.03.2004 GB 0405349
(43) Date of publication of application: 27.12.2006
(73) Proprietor: THE UNIVERSITY OF BIRMINGHAM, Birmingham B15 2TT (GB)
(72) Inventor: CAMPBELL, Moray James, Inst. of Biomedical Res., Edgbaston, Birmingham B15 2TT (GB); BUNCE, Christopher, School of Bioscience, Birmingham B15 2TT (GB); GOMMERSALL, Lyndon, Inst. of Biomedical Res., Edgbaston, Birmingham B15 2TT (GB); PEEHL, Donna, Department of Urology, Stanford, CA 94305-5118 (US)
(74) Representative: Ward, David Ian
(86) International application number: PCT/GB2005/000938
(87) International publication number: WO 2005/087206

(56) References cited:
- WO-A-99/37150
- WO-A-02/060430
- TABE YOKO ET AL: "Effects of Histone Deacetylase Inhibitor Suberoylanikide Hydroxamic Acid (SAHA) and DNA Methylation Inhibitor 5-aza-2'-deoxycytidine (DAC) on the Transcriptional Activation of RARbeta and p21WAF in Acute Promyelocytic Leukemia Cells." BLOOD, vol. 100, no. 11, 16 November 2002 (2002-11-16), page Abstract No. 3028, XP009054007 & 44TH ANNUAL MEETING OF THE AMERICAN SOCIETY OF HEMATOLOGY; PHILADELPHIA, PA, USA; DECEMBER 06-10, 2002 ISSN: 0006-4971
- CHANG TSG-HUI ET AL: "Enhanced growth inhibition by combination differentiation therapy with ligands of peroxisome proliferator-activated receptor-gamma and inhibitors of histone deacetylase in adenocarcinoma of the lung." CLINICAL CANCER RESEARCH : AN OFFICIAL JOURNAL OF THE AMERICAN ASSOCIATION FOR CANCER RESEARCH. APR 2002, vol. 8, no. 4, April 2002 (2002-04), pages 1206-1212, XP002346008 ISSN: 1078-0432
- KOSUGI HIROSHI ET AL: "In vivo effects of a histone deacetylase inhibitor, FK228, on human acute promyelocytic leukemia in NOD/Shi-scid/scid mice" JAPANESE JOURNAL OF CANCER RESEARCH, JAPANESE CANCER ASSOCIATION, TOKYO, JP, vol. 92, no. 5, 31 May 2001 (2001-05-31), pages 529-536, XP002960284 ISSN: 0910-5050
- HE L Z ET AL: "Transcription therapy for cancer: Effects of histone deacetylase (HDAC) inhibitors on proliferation, apoptosis and differentiation of leukemia cell lines" BLOOD, W.B. SAUNDERS, PHILADELPHIA, VA, US, vol. 92, no. 10 SUPPL 1 PART 1-2, 15 November 1998 (1998-11-15), page 510A, XP001030764 ISSN: 0006-4971
- KUENDGEN A ET AL: "Valproic acid alone and in combination with all-trans retinoic acid (ATRA) for the treatment of myelodysplastic syndromes and sAML/MDS." LEUKEMIA RESEARCH, vol. 27, no. Supplement 1, May 2003 (2003-05), page S104, XP002346009 & 7TH INTERNATIONAL SYMPOSIUM ON MYELODYSPLASTIC SYNDROMES; PARIS, FRANCE; MAY 15-18, 2003 ISSN: 0145-2126
- GRAZIANI GRAZIA ET AL: "Valproic acid increases the stimulatory effect of estrogens on proliferation of human endometrial adenocarcinoma cells." ENDOCRINOLOGY, vol. 144, no. 7, July 2003 (2003-07), pages 2822-2828, XP002346010 ISSN: 0013-7227
- RASHID SAMANTHA F ET AL: "Synergistic growth inhibition of prostate cancer cells by 1alpha,25 Dihydroxyvitamin D3 and its 19-nor-hexafluoride analogs in combination with either sodium butyrate or trichostatin A" ONCOGENE, vol. 20, no. 15, 5 April 2001 (2001-04-05), pages 1860-1872, XP002346011 ISSN: 0950-9232 cited in the application
- KOURAKLIS G ET AL: "Histone deacetylase inhibitors and anticancer therapy." CURRENT MEDICINAL CHEMISTRY - ANTI-CANCER AGENTS, vol. 2, no. 4, July 2002 (2002-07), pages 477-484, XP009054064 ISSN: 1568-0118

### SEQUENCE LISTING

<110> university of Birmingham
   Campbell, Moray J.
   Bunce, Christopher
   Gommersall, Lyndon
   Peehl, Donna
<120> CANCER THERAPY AND MEDICAMENTS THEREFOR
<130> W072467PPC
<140> GB05/000938 <141> 2005-03-10
<150> GB0405349.2 <151> 2004-03-10
<160> 17
<170> PatentIn version 3.1
<210> 1
   <211> 23
   <212> DNA
   <213> Artificial Sequence
<220>
   <223> GADD45a Forward primer
<400> 1
   aagaccgaaa ggatggataa ggt 23
<210> 2
   <211> 19
   <212> DNA
   <213> Artificial Sequence
<220>
   <223> GADD45a Reverse primer
<400> 2
   gtgatcgtgc gctgactca 19
<210> 3
   <211> 24
   <212> DNA
   <213> Artificial Sequence
<220>
   <223> GADD45a Probe
<400> 3
   tgctgagcac ttcctccagg gcat 24
<210> 4
   <211> 23
   <212> DNA
   <213> Artificial Sequence
<220>
   <223> Alien Forward primer
<400> 4
   cctcatccac tgattatggg agt 23
<210> 5
   <211> 23
   <212> DNA
   <213> Artificial Sequence
<220>
   <223> Alien Reverse primer
<400> 5
   catcataatt cttgaaggct tca 23
<210> 6
   <211> 31
   <212> DNA
   <213> Artificial Sequence
<220>
   <223> Alien Probe
<400> 6
   ccctcaagtg cattttacca ccacattctc t 31
<210> 7
   <211> 20
   <212> DNA
   <213> Artificial Sequence
<220>
   <223> NCoR1Forward primer
<400> 7
   tgaaggtctt ggcccaaaag 20
<210> 8
   <211> 23
   <212> DNA
   <213> Artificial sequence
<220>
   <223> NCoR1Reverse primer
<400> 8
   tttgtcttga tgttctcatg gta 23
<210> 9
   <211> 31
   <212> DNA
   <213> Artificial Sequence
<220>
   <223> NCoR1Probe
<400> 9
   ctgccactgt ataaccagcc atcagatacc a 31
<210> 10
   <211> 20
   <212> DNA
   <213> Artificial Sequence
<220>
   <223> SMRT Forward primer
<400> 10
   cacccggcag tatcatgaga 20
<210> 11
   <211> 20
   <212> DNA
   <213> Artificial Sequence
<220>
   <223> SMRT Reverse primer
<400> 11
   cgagcgtgat tcctcctctt 20
<210> 12
   <211> 22
   <212> DNA
   <213> Artificial Sequence
<220>
   <223> SMRT Probe
<400> 12
   cttccgcatc gcctggttta tt 22
<210> 13
   <211> 20
   <212> DNA
   <213> Artificial Sequence
<220>
<223> VDR Forward Primer
<400> 13
   cttcaggcga agcatgaagc 20
<210> 14
   <211> 20
   <212> DNA
   <213> Artificial Sequence
<220>
   <223> VDR Reverse Primer
<400> 14
   ccttcatcat gccgatgtcc 20
<210> 15
   <211> 26
   <212> DNA
   <213> Artificial sequence
<220>
   <223> VDR Probe
<400> 15
   aaggcactat tcacctgccc cttcaa 26
<210> 16
   <211> 46
   <212> DNA
   <213> Artificial Sequence
<220>
   <223> Primer containing T7 Promoter Sequence
<400> 16
   gcgtaatacg actcactata gggagacggg ctcctggagt accagc 46
<210> 17
   <211> 46
   <212> DNA
   <213> Artificial sequence
<220>
   <223> Primer containing T7 Promoter sequence
<400> 17
   gcgtaatacg actcactata gggagagctc cacctggggc cccagg 46

## Description

The present invention relates in one aspect to a novel cancer therapy, particularly but not exclusively a prostate, bladder and breast cancer therapy and to compositions and medicaments for use in said therapy. In another aspect, the invention provides a method of reducing proliferation of and/or inducing programmed cell death (e.g. apoptosis) in neoplastic cells.

The nuclear receptor (NR) superfamily is a large family (48 human members) of ligand-activated transcription factors which collectively regulate multiple aspects of proliferation and differentiation. The NRs can be subdivided into
- Classical endocrine receptors that bind ligands with high affinity, e.g., androgen receptor (AR), oestrogen receptors (ERs), vitamin D receptor (VDR)
- Adopted orphan receptors that bind ligands with broader affinity e.g., Peroxisome proliferator activated receptors (PPARs), bile acid receptor (FXR), Lipid X receptors (LXRs) and pregnane X receptor (PXR).
- Orphan receptors, for which no ligand has yet been identified or exists.

NR actions are disrupted in cancer. It has previously been demonstrated that aggressive androgen independent prostate cancer cell lines often display reduced sensitivity to the antiproliferative action of a variety of nuclear receptor ligands such as those for the RARs (retinoic acid receptors), PPAR and VDR. Initial studies to investigate mechanisms which disrupt NR antiproliferative action focused on VDR as a key member of the family which responds to environmental signals and has a strong association with prostate cancer. Proliferation and differentiation of normal prostate epithelial cells is acutely regulated *in vitro* and *in vivo* by 1α,25-dihydroxyvitamin D₃ (1α,25(OH)₂D₃, high affinity ligand for VDR) which transactivates a number of anti-proliferative target genes (including the CDKI p21^{*(wafI*/*cipI)*}), thereby justifying clinical trials in prostate cancer patients. However, the antiproliferative response is reduced to various degrees in some prostate tumours. Multiple epidemiological studies have now linked the incidence of prostate cancer to low serum levels of the 1α,25(OH)₂D₃ precursor, 25(OH)D₃ as a result of either diet or environment. Furthermore, certain VDR polymorphisms are associated with elevated incidence. Collectively, such data link initiation or progression of prostate cancer with reduced dietary intake and/or cellular resistance to the antiproliferative effects of 1α,25(OH)₂D₃.

The NRs share a common architecture to act as transcription factors including defined regions for DNA recognition, ligand binding and cofactor interactions. The DNA-binding domain recognises specific nucleotide sequences (response elements). Ligand binding induces conformational changes that switch NR binding from co-repressor (CoR) proteins e.g. NCoR2/SMRT and NCoR1 to co-activator proteins (CoA). Associated with these CoA or CoR proteins are histone acetyltransferases (HAT) and histone deacetylases (HDAC), respectively, which together form part of large multimeric gene activation or repression complexes. One function of these complexes is to regulate a range of post-translational modifications of histone tails by a variety of processes including acetylation, methylation and phosphorylation, thereby regulating the affinity to DNA. Repression complexes with HDAC activity induce local decondensation of chromatin and repress gene activation.

Differences in tissue-specific NR actions have been attributed to altered CoA/CoR expression patterns, for example the cell-type specific actions of the ERα partial antagonist, tamoxifen is dependent upon CoA expression. Thus cellular sensitivity to NR action is strongly influenced by CoA/CoR expression. Furthermore these interactions are disrupted in malignancy. For example a variety of studies have demonstated that NR sensitivity to ligand is dysregulated in myeloid leukaemia and breast cancer and, by alterations to either CoA or CoR activity.

One of the best known chromosomal translocations involving nuclear receptors in carcinogenesis occurs in acute promyelocytic leukaemia. The causative translocation in most cases occurs between chromosomes 15 and 17 creating a fusion product between the PML and the RARα genes. The resulting fusion protein (PML- RARα) has a higher affinity for the CoR NCoR1 and therefore inappropriately retains histone deacetylases around the responsive regions of RARα target gene promoters and leads to abnormal silencing of the normally pro-differentiating retinoid signalling. Also, the fusion protein ablates the normal functions of the PML protein, which include co-activation of the p53 tumour suppressor gene and promotion of apoptosis (Altucci, L. and Gronemeyer, H. Nuclear receptors in cell life and death. Trends Endocrinol. Metab, 12: 460-468, 2001.) (Altucci L, Nature Reviews Cancer 2001). Similarly in breast cancer the CoA AIB1 is well described for being overexpressed and thereby resulting in inappropriately enhanced ERα signalling (Anzick, S. L., Kononen, J., Walker, R. L., Azorsa, D. O., Tanner, M. M., Guan, X. Y., Sauter, G., Kallioniemi, O. P., Trent, J. M., and Meltzer, P. S. AIB1, a steroid receptor coactivator amplified in breast and ovarian cancer. Science, 277: 965-968, 1997.).

The molecular mechanisms for 1α,25(OH)₂D₃-insensitivity in prostate cancer are as yet unclear. It has previously been demonstrated that the VDR is neither mutated nor have receptor expression studies established a clear relationship between VDR content and antiproliferative effect by 1α,25(OH)₂D₃. Indeed, PC-3 and DU 145 prostate cancer cell lines are relatively 1α,25(OH)₂D₃-insensitive and yet VDR transactivation is sustained and even enhanced, as measured by induction of CYP24 gene, a highly inducible VDR target gene. Previously the inventors have shown that co-treatment of prostate cancer cell lines (LNCaP, PC-3 and DU 145) with 1α,25(OH)₂D₃ plus either trichostatin A (TSA) or sodium butyrate (NaB) as an HDAC inhibitor, resulted in synergistic growth inhibition and induction of apoptosis although the targets of gene activation remained unclear (Rashid, S. F. et al. Synergistic growth inhibition of prostate cancer cells by 1α,25Dihydroxyvitamin D3 and its 19-nor-hexafluoride analogs in combination with either sodium butyrate or trichostatin A. Oncogene 20, 1860-1872 (2001)).

It is an object of the present invention in one aspect to provide a novel cancer therapy and a medicament or combination of medicaments for use in said therapy.

According to a first aspect of the present invention there is provided the use of a therapeutically effective amount of a nuclear receptor ligand and an HDAC inhibitor in the manufacture of a medicament or respective medicaments for the treatment of cancer, characterized in that said nuclear receptor ligand is a PPAR ligand selected from bezafibrate, EPA and ETYA or an FXR ligand selected from CDA and LCA, and said HDAC inhibitor is SAHA.

The nuclear receptor ligand and HDAC inhibitor may be administered sequentially, concomitantly or combined as a single medicament.

The present invention also resides in the use of said nuclear receptor ligand and said HDAC inhibitor in the manufacture of a medicament for the reduction or prevention of proliferation of neoplastic cells or for the induction of programmed cell death (eg. apoptosis) in said neoplastic cells, or in the manufacture of respective medicaments for concomitant or sequential administration.

According to another aspect of the present invention there is provided a method of reducing proliferation and/or inducing programmed cell death of in vitro neoplastic cells exhibiting abnormal expression or activity of a co-repressor protein, comprising contacting said cells with said HDAC inhibitor and an anti-proliferative and/or programmed cell death-inducing gene trans-activating factor, whereby to induce expression of said anti-proliferative and/or programmed-cell death-inducing gene.

According to another aspect of the present invention, there is provided a synergistic combination of said HDAC inhibitor and said nuclear receptor ligands, for reducing proliferation of or inducing programmed cell death in neoplastic cells.

The present invention also resides in the use of a nuclear receptor ligand as a synergist in combination with an HDAC inhibitor in the manufacture of a medicament for the treatment of cancer, said nuclear receptor ligands being selected from ligands for the PPAR, and FXR receptors, more preferably the PPAR receptor and most preferably PPARα and PPARγ.

The following statements relate to preferred modes of the invention, unless explicitly excluded in any specific aspects of the invention above.

The nuclear receptor ligand/anti-proliferative gene trans-activating factor are chenodeoxycholic acid (CDA)), bezafibrate (BF), eicosapentaenoic acid (EPA), 5,8,11,14-eicosatetraenoic acid (ETYA) and lithocholic acid (LCA).

The HDAC inhibitor is suberoylanilide hydroxamic acid (SAHA).

Preferably, the anti-proliferative and/or programmed cell death-inducing gene is one of Id-1H, cyclin K, MAPK-APK2, p21-rac1, p21^{*(waf1*/*cip1)*}, zyxin, ZO-1, VDUP-1, GADD45, CTNNB, VE-cadherin, CYP3A4 and EMAP II, most preferably MAPK-APK2 and GADD45α.

Preferably, the co-repressor protein is selected from NCoR2/SMRT, NCoR1 and TRIP15/Alien and is more preferably NCoR2/SMRT and NCoR1.

Two particularly effective combinations are
(i) SAHA and a PPAR ligand such as bezafibrate, EPA or ETYA, and
(ii) SAHA and an FXR ligand such as CDA and LCA.

The therapies, methods and medicaments of the present invention may be particularly useful in the treatment of prostate, bladder, oesophageal, breast, lung and colonic cancers and myeloid leukaemia, and most especially bladder and prostate cancers.

The therapies, methods and medicaments of the present invention are particularly useful in the treatment of tumours which show a reduced response relative to normal tissue to nuclear receptor ligands/trans-activating factors used alone.

The neoplastic cells may be epithelial cells, particularly epithelial cells from prostate, bladder, colon, breast and squamous and normal myeloid progenitors.

The dosage administered to a patient will normally be determined by the prescribing physician and will generally vary according to the age, weight and response of the individual patient, as well as the severity of the patient's symptoms. However, in most instances, an effective therapeutic daily dosage will be that which is sufficient to deliver an extracellular concentration of HDAC inhibitor of 0.2 µM or more, preferably 0.4 µM or more and most preferably between about 0.5 and 2 µM and an extracellular concentration of nuclear receptor ligand of 0.05 µM or more, preferably 0.1 µM or more and preferably no more than about 2 µM administered in single or divided doses. In some cases, however, it may be necessary to use dosages outside these limits.

While it is possible for an active ingredient to be administered alone as the raw chemical, it is preferable to present it as a pharmaceutical formulation. The formulations, both for veterinary and for human medical use, of the present invention comprise the active ingredient(s) in association with a pharmaceutically acceptable carrier therefor and optionally other therapeutic ingredient(s). The carrier(s) must be 'acceptable' in the sense of being compatible with the other ingredients of the formulation and not deleterious to the recipient thereof.

Preferably, the formulation is suitable for administration from one to six, such as two to four, times per day and unit doses can be made up accordingly. For topical administration, the active ingredient preferably comprises from 1% to 2% by weight of the formulation but the active ingredient may comprise as much as 10% w/w. Formulations suitable for nasal or buccal administration, such as the self-propelling powder-dispensing formulations described hereinafter, may comprise 0.1 to 20% w/w, for example about 2% w/w of active ingredient.

The formulations include those in a form suitable for oral, ophthalmic, rectal, parenteral (including subcutaneous, vaginal, intraperitoneal, intramuscular and intravenous), intra-articular, topical, nasal or buccal administration.

Formulations of the present invention suitable for oral administration may be in the form of discrete units such as capsules, cachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. The active ingredient may also be in the form of a bolus, electuary or paste. For such formulations, a range of dilutions of the active ingredient in the vehicle is suitable, such as from 1% to 99%, preferably 5% to 50% and more preferably 10% to 25% dilution. Depending upon the level of dilution, the formulation will be either a liquid at room temperature (in the region of about 20°C) or a low-melting solid.

Formulations for rectal administration may be in the form of a suppository incorporating the active ingredient and a carrier such as cocoa butter, or in the form of an enema.

Formulations suitable for parenteral administration comprise a solution, suspension or emulsion, as described above, conveniently a sterile aqueous preparation of the active ingredient that is preferably isotonic with the blood of the recipient.

Formulations suitable for intra-articular administration may be in the form of a sterile aqueous preparation of the active ingredient, which may be in a microcrystalline form, for example, in the form of an aqueous microcrystalline suspension or as a micellar dispersion or suspension. Liposomal formulations or biodegradable polymer systems may also be used to present the active ingredient particularly for both intra-articular and ophthalmic administration.

Formulations suitable for topical administration include liquid or semi-liquid preparations such as liniments, lotions or applications; oil-in-water or water-in-oil emulsions such as creams, ointments or pastes; or solutions or suspensions such as drops. For example, for ophthalmic administration, the active ingredient may be presented in the form of aqueous eye drops, as for example, a 0.1-1.0% solution.

Drops according to the present invention may comprise sterile aqueous or oily solutions. Preservatives, bactericidal and fungicidal agents suitable for inclusion in the drops are phenylmercuric salts (0.002%), benzalkonium chloride (0.01%) and chlorhexidine acetate (0.01%). Suitable solvents for the preparation of an oily solution include glycerol, diluted alcohol and propylene glycol.

Lotions according to the present invention include those suitable for application to the eye. An eye lotion may comprise a sterile aqueous solution optionally containing a bactericide or preservative prepared by methods similar to those for the preparation of drops. Lotions or liniments for application to the skin may also include an agent to hasten drying and to cool the skin, such as an alcohol, or a softener or moisturiser such as glycerol or an oil such as castor oil or arachis oil.

Creams, ointments or pastes according to the present invention are semi-solid formulations of the active ingredient in a base for external application. The base may comprise one or more of a hard, soft or liquid paraffin, glycerol, beeswax, a metallic soap; a mucilage; an oil such as a vegetable oil, eg almond, corn, arachis, castor or olive oil; wool fat or its derivatives; or a fatty acid ester of a fatty acid together with an alcohol such as propylene glycol or macrogols. The formulation may also comprise a suitable surface-active agent, such as an anionic, cationic or non-ionic surfactant such as a glycol or polyoxyethylene derivatives thereof. Suspending agents such as natural gums may be incorporated; optionally with other inorganic materials, such as silicaceous silicas, and other ingredients such as lanolin.

Formulations suitable for administration to the nose or buccal cavity include those suitable for inhalation or insufflation, and include powder, self-propelling and spray formulations such as aerosols and atomisers. The formulations, when dispersed, preferably have a particle size in the range of 10 to 200µ.

Such formulations may be in the form of a finely comminuted powder for pulmonary administration from a powder inhalation device or self-propelling powder-dispensing formulations, where the active ingredient, as a finely comminuted powder, may comprise up to 99.9% w/w of the formulation.

Self-propelling powder-dispensing formulations preferably comprise dispersed particles of solid active ingredient, and a liquid propellant having a boiling point of below 18° C at atmospheric pressure. Generally, the propellant constitutes 50 to 99.9% w/w of the formulation whilst the active ingredient constitutes 0.1 to 20% w/w. for example, about 2% w/w, of the formulation.

The pharmaceutically acceptable carrier in such self-propelling formulations may include other constituents in addition to the propellant, in particular a surfactant or a solid diluent or both. Surfactants are desirable since they prevent agglomeration of the particles of active ingredient and maintain the active ingredient in suspension. Especially valuable are liquid non-ionic surfactants and solid anionic surfactants or mixtures thereof. Suitable liquid non-ionic surfactants are those having a hydrophile-lipophile balance (HLB, see Journal of the Society of Cosmetic Chemists Vol. 1 pp. 311-326 (1949)) of below 10, in particular esters and partial esters of fatty acids with aliphatic polyhydric alcohols. The liquid non-ionic surfactant may constitute from 0.01 up to 20% w/w of the formulation, though preferably it constitutes below 1% w/w of the formulation. Suitable solid anionic surfactants include alkali metal, ammonium and amine salts of dialkyl sulphosuccinate and alkyl benzene sulphonic acid. The solid anionic surfactants may constitute from 0.01 up to 20% w/w of the formulation, though preferably below 1% w/w of the composition. Solid diluents may be advantageously incorporated in such self-propelling formulations where the density of the active ingredient differs substantially from the density of the propellant; also, they help to maintain the active ingredient in suspension. The solid diluent is in the form of a fine powder, preferably having a particle size of the same order as that of the particles of the active ingredient. Suitable solid diluents include sodium chloride, sodium sulphate and sugars.

Formulations of the present invention may also be in the form of a self-propelling formulation wherein the active ingredient is present in solution. Such self-propelling formulations may comprise the active ingredient, propellant and co-solvent, and advantageously an antioxidant stabiliser. Suitable co-solvents are lower alkyl alcohols and mixtures thereof. The co-solvent may constitute 5 to 40% w/w of the formulation, though preferably less than 20% w/w of the formulation. Antioxidant stabilisers may be incorporated in such solution-formulations to inhibit deterioration of the active ingredient and are conveniently alkali metal ascorbates or bisulphites. They are preferably present in an amount of up to 0.25% w/w of the formulation.

Formulations of the present invention may also be in the form of an aqueous or dilute alcoholic solution, optionally a sterile solution, of the active ingredient for use in a nebuliser or atomiser, wherein an accelerated air stream is used to produce a fine mist consisting of small droplets of the solution. Such formulations usually contain a flavouring agent such as saccharin sodium and a volatile oil. A buffering agent such as sodium metabisulphite and a surface-active agent may also be included in such a formulation which should also contain a preservative such as methylhydroxybenzoate.

Other formulations suitable for nasal administration include a powder, having a particle size of 20 to 500 microns, which is administered in the manner in which snuff is taken, *i*.*e*. by rapid inhalation through the nasal passage from a container of the powder held close up to the nose.

In addition to the aforementioned ingredients, the formulations of this invention may include one or more additional ingredients such as diluents, buffers, flavouring agents, binders, surface active agents, thickeners, lubricants, preservatives e.g. methylhydroxybenzoate (including antioxidants), emulsifying agents and the like. A particularly preferred carrier or diluent for use in the formulations of this invention is a lower alkyl ester of a C₁₈ to C₂₄ mono-unsaturated fatty acid, such as oleic acid, for example ethyl oleate. Other suitable carriers or diluents include capric or caprylic esters or triglycerides, or mixtures thereof, such as those caprylic/capric triglycerides sold under the trade name Miglyol, *e.g.* Miglyol 810.

The invention will be further described by way of example only with reference to the accompanying drawings in which:-
Figure 1A is a graph of NCoR2/SMRT levels for normal prostate and primary prostate tumours,
Figure 1B is a graph of NCoR2/SMRT, NCoR1, TRIP15/Alien in breast cancer cell lines,
Figure 1C is a graph of NCoR1 expression tumour biopsies compared to matched normal,
Figures 2A to E are plots of proliferation in various cell lines after treatment with different combinations of nuclear receptor ligand and HDAC inhibitor,
Figures 3A and 3B are graphs of cell proliferation as measured by ATP bioluminescence for SAHA alone or in combination with 1α,25(OH)₂D₃ and bezafibrate respectively, measured as a percentage relative to control,
Figure 4 is a graph of EJ-28 bladder cancer cells treated with a range of nuclear receptor ligands alone and in combination with SAHA,
Figures 5A and 5B are graphs of cell proliferation as measured by ATP bioluminescence for SAHA alone or in combination with EPA and ETYA respectively, measured as a percentage relative to control,
Figure 6 is a plot of cell cycle data for Du145 cells after various treatments,
Figure 7A is a plot of SMRT mRNA expression before and after knock down, and
Figure 7B is a plot of GADD45α induction upon treatment with 1α,25(OH)₂D₃ or bezafibrate.

### Materials and Methods

### Nuclear receptor ligands and HDAC inhibitors

A range of clinically-applicable NR ligands were investigated:-
all *trans* retinoic acid (ATRA) which binds to RAR/RXR, 9-cis retinoic acid (9cisRA) which binds to RXR, chlofibric acid (CA), bezafibrate (BF) Eicosapentaenoic acid (EPA) and 5,8,11,14-eicosatetraenoic acid (ETYA) which bind to PPARα and γ, medroxy progesterone acetate (MPA) which binds to ER/PR, and Thyroid T₃ (which binds the TR), 1α,25(OH)₂D₃ which binds to VDR, chenodeoxycholic acid (CDA) which binds to the FXR, and lithocholic acid (LCA) which binds FXR and VDR.

The 1α,25(OH)₂D₃ (generous gift of Dr. Milan R. Uskokovic, Hoffman La Roche, Nutley, NJ 07110, U.S.A.) and TSA (Sigma, Poole, U.K.) were stored as 1 mM stock solutions in ethanol at -20°C. Suberoylanilide hydroxamic acid (SAHA) was supplied by ATON Pharma (New York, US) and stored as a 100 mM stock solution in DMSO. Other nuclear receptor ligands were purchased from Sigma and diluted and stored according to the manufacturers' instructions

### Cell culture

Normal prostate epithelial cells (PrEC) were cultured in PrEGM media (Clonetics, Wokingham, UK) according to manufacturer's instructions. The prostate and bladder cancer cell lines were obtained from the American Type Culture Collection (ATCC, Rockville, MD) and cultured according to the guidance of ATCC. Cells were passaged by trypsinising with 0.25% trypsin-EDTA (Gibco-BRL). All cells were grown at 37°C in a humidified atmosphere of 5% CO₂ in air.

### Primary Prostate cultures

Tissues dissected from radical prostatectomy specimens were processed for primary culture of prostatic epithelial cells according to previously described methods (Peehl DM Are primary cultures realistic models of prostate cancer? J. Cell Biochem. 91 185-195 (2004)). None of the patients had received prior chemical, hormonal or radiation therapy. Each cell strain was serially passaged and cells in secondary or tertiary passages were used for clonal growth assays or RNA isolation.

### Primary breast material (mRNA from cell lines and tumour panels)

Matched tumour and normal tissue (a generous gift from Dr. Kay Colston, St. George's Hopsital London) and was obtained from biopsies/resection specimens of Caucasian female patients who had undergone surgery for invasive ductal breast cancer at St. George's Hospital, London, UK. Age of diagnosis of primary tumour (range 35-88) and oestrogen receptor (ER) status were validated from histopathological reports and patient medical. The study received local ethical approval from St. George's Hospital Medical School Ethics Committee. Total RNA was extracted using the RNeasy and the Lipid Tissue Mini Kit (Qiagen, UK). Briefly, a piece of breast tissue, approximately 2mm³ in size, was excised from the relevant frozen surgical sample, which had been stored in liquid nitrogen. The tissue was placed directly into 1ml of lysis reagent and homogenised using a rotor-stator homogeniser (IKA-WERKE, Germany). RNA was then extracted according to the manufacturer's instructions, with one modification; before ethanol washes, DNA digestion was carried out using RQ1 Rnase-Free DNase (Promega, UK) by the addition of 10µl RQ1 Dnase, 10µl of 10X RQ1 buffer, and 80µl H2O to each column, followed by 15 minutes incubation at room temperature. RNA was eluted in 30 µl of RNase-free water and stored at -70°C.

Aliquots of mRNA from MDA-134, MDA-361, BT-474, MDA-175, MDA-468, BT-20, HBL100, HMT3532, ZR-75-6, GZ101, BMC42, CAZ51, SXBR5, SXBR6, were supplied by Dr. Kay Colston (St. George's Hosptial, London)

### Proliferation assays

The action of individual agents alone and in combination was examined using a bioluminescent technique to measure changes in cellular ATP (ViaLight HS, LumiTech, Nottingham, U.K.) with previously optimised conditions according to the manufacturer's instructions (Rashid, S. F. *et al. Supra*). Briefly, cells were plated in 96 well white-walled tissue culture-treated plates (Fisher Scientific Ltd. Loughborough, U.K.) (cancer cell lines at 2 x 10³ cells/well; PrEC at 3.5 x 10³ cells/well). Growth media containing varying concentrations of SAHA, TSA, 1α,25(OH)₂D₃, nuclear receptor ligands were added to a final volume of 100 µl/well and plates were incubated for 96 h, with re-dosing after 48 h. After the incubation period, 100 µl of nucleotide releasing reagent was added to each well and cells were left for 30 minutes at room temperature. Liberated ATP was quantitated by adding 20 µl of ATP monitoring reagent (containing luciferin and luciferase) and measuring luminescence with a microplate luminometer (Berthold Detection Systems, Fisher Scientific Ltd.). ATP levels were recorded in relative luciferase units and growth inhibition was expressed as a percentage of control.

Clonal growth assays were used to evaluate responses of primary cultures to 1α,25(OH)₂D₃. Each 60-mm tissue culture dish, coated with collagen and containing 5 ml of serum-free medium and vehicle or 1α,25(OH)₂D₃, was inoculated with 500 cells per dish. After 10 days of incubation, cells were fixed with formalin and stained with crystal violet. Total cell growth was measured with an Artek image analyser (Dynatech, Chantilly, VA, USA). All experiments were performed three times and in triplicate.

### Cell Cycle Analysis

The effect of nuclear receptor ligands in combination with the HDAC inhibitors on the cell cycle was measured by staining DNA with propidium iodide (PI). Briefly, T25 flasks were seeded with 2.5x10⁵ sub-confluent, exponentially proliferating cells, exposed to 100 nM 1α,25(OH)₂D₃ alone or combined with either 300 µM NaB or 15nM TSA at times 0 (and re-dosed after 48 hours in 72 and 96 hr assays). After a total of 24, 48, 72 and 96 hours, cultures were harvested by trypsinising, counted and 1x10⁶ cells were stained with PI buffer (10µg/ml PI, 1%(w/v) tri-sodium citrate, 0.1%(v/v) Triton X-100, 100µM sodium chloride (Sigma)). Cell cycle distribution was determined using a Becton-Dickinson Flow Cytometer and CellFIT Cell-Cycle Analysis software.

### Extraction of RNA and reverse transcription

Cells were seeded at a density of 2x10⁴/cm² and allowed to grow for 36 h to ensure that cells were in mid-exponential phase upon treatment. Total RNA was extracted using the GenElute RNA extraction system (Sigma) according to manufacturer's instructions. Primary cultures were serially passaged and grown to 80% confluency in standard serum-free medium. Cells were fed one day prior to isolation of total RNA using the Qiagen RNeasy Midi kit (Qiagen, Palo Alto, CA, USA).

For real time reverse transcription-polymerase chain reaction (RT-PCR), cDNA was prepared from 1 µg of total RNA by reverse transcription with Mu-MLV (Promega Southampton, UK) at 42°C for 60 min in the presence of 100 mM Tris-HCl, pH 9.0, 500 mM KCl and 2 mM MgCl₂, 100 pM random hexamers (Pharmacia, Pisacataway, NJ), 2 mM dNTP and 20 U RNAsin (Promega) in a 20 µl reaction volume.

### Real-Time Quantitative RT-PCR (Q-RT-PCR)

Expression of specific mRNAs was quantitated using the ABI PRISM 7700 Sequence Detection System. Each sample was amplified in triplicate wells in 25 µl volumes containing lx TaqMan Universal PCR Master Mix [3 mM Mn(OAc)₂, 200 µM dNTPs, 1.25 units AmpliTaq Gold polymerase, 1.25 units AmpErase UNG], 3.125 *p*moles FAM-labelled TaqMan probe and 22.5 *p*moles primers. All reactions were multiplexed with pre-optimised control primers and VIC labelled probe for 18S ribosomal RNA (PE Biosystems, Warrington, UK). Primer and probe sequences are given in Table 1. Reactions were cycled as follows: 50° C for 2 min, 95° C for 10 min; then 44 cycles of 95° C for 15 sec and 60°C for 1 min.

Data were expressed as Ct values (the cycle number at which logarithmic PCR plots cross a calculated threshold line) and used to determine δCt values (δCt = Ct of the target gene minus Ct of the housekeeping gene). The data was transformed through the equation 2^{-δδCc} to give fold changes in gene expression. To exclude potential bias due to averaging of data all statistics were performed with δCt values. Measurements were carried out a minimum of three times each in triplicate wells for cell lines and once each in triplicate wells for primary material.

**Table 1: Primer and Probe Sequences used in Q-RT-PCR**

| **Primer** | **Sequence** |
|---|---|
| GADD45α Forward primer | AAGACCGAAAGGATGGATAAGGT |
| GADD45α Reverse primer | GTGATCGTGCGCTGACTCA |
| GADD45α Probe | TGCTGAGCACTTCCTCCAGGGCAT |
| Alien Forward primer | CCTCATCCACTGATTATGGGAGT |
| Alien Reverse primer | CATCATAATTCTTGAAGGCTTCA |
| Alien Probe | CCCTCAAGTGCATTTTACCACCACATTCTCT |
| NCoR1Forward primer | TGAAGGTCTTGGCCCAAAAG |
| NCoR1Reverse primer | TTTGTCTTGATGTTCTCATGGTA |
| NCoR1Probe | CTGCCACTGTATAACCAGCCATCAGATACCA |
| SMRT Forward primer | CACCCGGCAGTATCATGAGA |
| SMRT Reverse primer | CGAGCGTGATTCCTCCTCTT |
| SMRT Probe | CTTCCGCATCGCCTGGTTTATT |
| VDR Forward Primer | CTTCAGGCGAAGCATGAAGC |
| VDR Reverse Primer | CCTTCATCATGCCGATGTCC |
| VDR Probe | AAGGCACTATTCACCTGCCCCTTCAA |

### Small interfering RNA to target SMRT.

SiRNA fragments (22mers) were generated *in vitro* using a Dicer generation system according to the manufacturer's protocol (Gene Therapy Systems, San Diego, CA). Briefly, a 650 bp region from the human SMRT gene sequence corresponding to 112 to 762 was amplified using the following primers containing T7 promoter sequences;
FS: 5' - gcgtaatacgactcactatagggagacgggctcctggagtaccagc - 3' and
RV: 5' - gcgtaatacgactcactatagggagagctccacctggggccccagg - 3'. This was subsequently cloned into the pGEM T easy vector (Promega), using the multiple cloning sites to allow for sequencing, large scale harvest. Digestion with ECoR1 released a pure concentrated template for *in vitro* translation with primers containing T7 recognition sequences to generate double stranded mRNA. This was subsequently cleaved with recombinant human Dicer enzyme to generate a pool of 22mers which cover the region targeted in the 5'prime region of the SMRT gene. Five hundred ng of purified double stranded RNA 22mers were transfected into each well (2x10⁵ cells/well in 24-well plates) for 12 hrs. Cells were then left for a further 72 hr to allow gene silencing and subsequently treated with 1α,25(OH)₂D₃ (100 nM) for 6 hr and total RNA was harvested and Q-RT-PCR for SMRT and target genes was undertaken as above.

### Statistical analysis

The interactions of two compounds were assessed by measuring the mean of either the nuclear receptor ligand or the HDAC inhibitor acting alone or in combination. The mean observed combined effect was compared to the individual effects of the agents added together, using the Student's *t*-test. Classification of the effects were as follows: strong additive (synergistic) effects were those with an experimental value significantly greater than the predicted value, additive effects were those where the experimental value did not significantly differ from the predicted value, sub-additive effects were those where the experimental value was significantly less than the predicated value.
All other analyses were also compared using the Student's t-test.

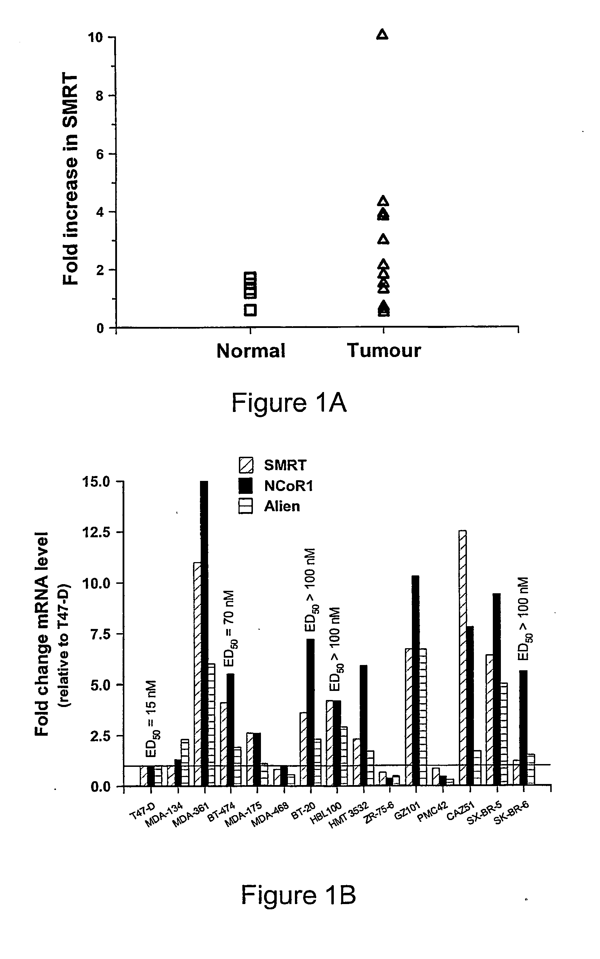

Co-repressors are elevated in prostate, breast and bladder cancer cells. Compared to PrEC cells there was significantly increased expression of NCoR2/SMRT in PC-3 and DU 145 cells (1.8 fold, (*p*< 0.05) and 2.2 fold, (*p*< 0.05) respectively). Of the PCa samples, #1243 (ED₅₀> 10nM) had clear elevated expression of NCoR2/SMRT, NCoR1 and TRIP15/Alien whereas #1241 predominantly expressed elevated NCoR2/SMRT only compared to levels in the peripheral zone cultures. Similarly in a panel of primary tumours we found elevated levels of NCoR2/SMRT for example 10/15 primary tumour culture samples had elevated NCoR2/SMRT mRNA levels (mean 4.2 fold increase); generally NCoR1 and TRIP 15/Alien were not as commonly elevated (3/15 and 2/15) (Figure 1A).

Taken together these data indicate that elevation of co-repressors, principally NCoR2/SMRT, is a common event and that it correlates with reduced responsiveness to nuclear receptor ligands.

These actions were investigated in bladder carcinoma cell lines, RT4 (papillary derived), RT112 (grade 2 derived) and HT1376 and EJ28 (high grade) by examining basal proliferation, invasion though Matrigel and antiproliferative responses to a range of nuclear receptors ligands. The high grade cell lines proliferated at the fastest rate, were the only ones to demonstrate significant and rapid invasion through Matrigel and 'healing' of a circular wound in a confluent monolayer culture. The cells also demonstrated a spectrum of responsiveness to the ligands; namely 1α,25(OH)₂D₃ and 9 *cis* retinoic acid (vitamin D receptor (VDR) and retinoic acid receptor (RAR)), the bile acids chenodeoxycholic acid and lithocholic acid (VDR and farnesoid receptor (FXR)), the omega 6 fatty acid 5,8,11,14-eicosatetraenoic acid (peroxisome proliferator activated receptors (PPARγ). Thus for example, RT-4 was inhibited by 1α,25(OH)₂D₃ (Vit D), 5,8,11,14-eicosatetraenoic acid (ETYA) and chenodeoycholic acid (CDA) with ED₅₀ of 80 nM, 6 µM and 11 µM respectively, whereas EJ-28 was insensitive to these treatments (Table 2).

**Table 2: Estimated dose of a range of nuclear receptor ligands and SAHA required to inhibit bladder cancer cell proliferation by 50% (ED50)**

| | Target receptor | **RT-4** | **HT-1376** | **RT-112** | **EJ-28** |
|---|---|---|---|---|---|
| **Vit D** | VDR | 80 nM | > 100 nM | 20 nM | > 100 nM |
| **9cis RA** | RXR | > 100 nM | > 100 nM | > 100 nM | > 100 nM |
| **ETYA** | PPARχ | 6 µM | 30 µM | 9 µM | 60 µM |
| **CDA** | FXR | 11 µM | 10 µM | 90 µM | > 100 nM |
| **LCA** | FXR/VDR | 20 µM | > 100 mM | 50 µM | > 100 nM |
| **SAHA** | - | 2 µM | 1 µM | 1 µM | 2 µM |

We have comprehensively profiled, by quantitative real time RT-PCR, expression levels of nuclear receptors (*VDR, PPAR*α, *PPAR*γ, *LXR*α, *LXR*β, *FXR*) co-repressors (*NCoR1, NCoR2*/*SMRT*, *Alien*) in these cells. These data suggested that suppressed cellular responsiveness to ligand reflect an altered ratio of receptor to co-repressor levels. Thus EJ-28 display reduced *PPAR*γ and FXR levels (0.03 and 0.0007 fold reduction) compared to RT-4, with comparable levels of TRIP 15/Alien and NCoR1 (Table 3)

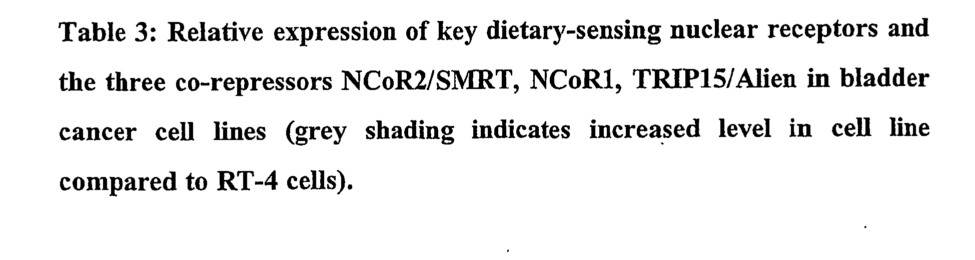

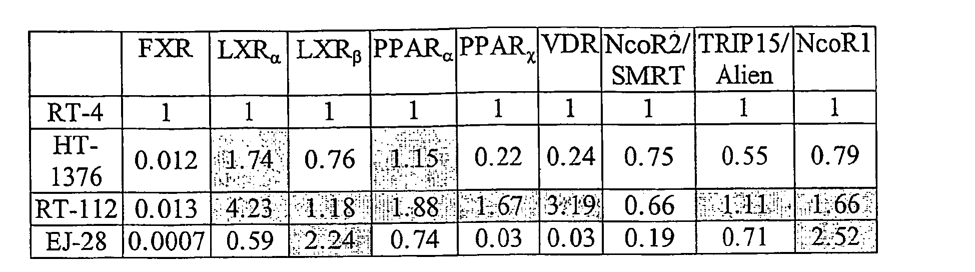

### NCoR1 is frequently elevated in breast cancer cell lines and primary matched tumour and normal material.

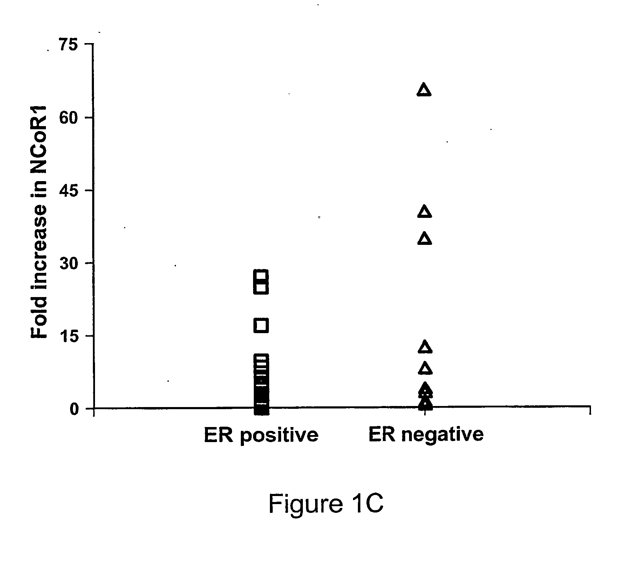

To investigate the significance of the elevation of co-repressors in the cell line panel we surveyed a broader panel of cell lines and a cohort of matched tumour and normal primary samples. The survey of 14 cell lines also revealed frequently elevated co-repressor mRNA. For these experiments we compared the cell lines to the T47-D cells, which we had established previously had comparable 1α,25(OH)₂D₃-sensitivity and levels of co-repressor to MCF-12A non-malignant cells. Ten of the cell lines had elevated (> 2 fold) increase in NCoR1 which appeared to correlate with loss of ERα staining as 2/4 of ERα positive cell lines had elevated levels (MDA-361, BT-4747) whereas 4/5 ERα negative cell lines had elevated levels (MDA-175, BT-20, HBL100, HMT3532). The ERα status of the remaining cell lines (GZ101, CAZ51, SK-BR-5 and SK-BR-6) with NCoR1 elevation was unknown. Furthermore in several cases this correlated with the established 1α,25(OH)₂D₃-insensitivity of the cell lines (Figure 1B). This was complemented by the examination of 32 matched tumour and normal samples taken from a range of patients with breast cancer. Several of the findings from cell line cultures are reflected in the tumour samples. Firstly, elevated NCoR1 levels (> 2 fold) were frequent in both ERα + ve (12/23) and ERα -ve (7/9) with a mean fold increase of 6.6± 1. 75 and 14± 6 respectively (Figure 1C); the higher expression in the ERα -ve cells reflecting the cell lines.

### Co-operative actions of SAHA and a range of nuclear receptor ligands in prostate and bladder cancer

SAHA alone potently and completely inhibits proliferation of common prostate cancer cell lines with ED₅₀ values in the range of 1 µM. At higher doses, for example 2 µM, we have demonstrated a potent and immediate upregulation of the CDKI p21^{(waf1/cip1)} associated with significant G₁ cell cycle arrest after 24 hr (e.g. DU-145 ctrl cells 41 % in G₁, SAHA treated cells (1 µM) 64 % in G₁), similar data was recorded in PC-3 cells and these responses were associated with type 2 non-apoptotic programmed cell death (data not shown).

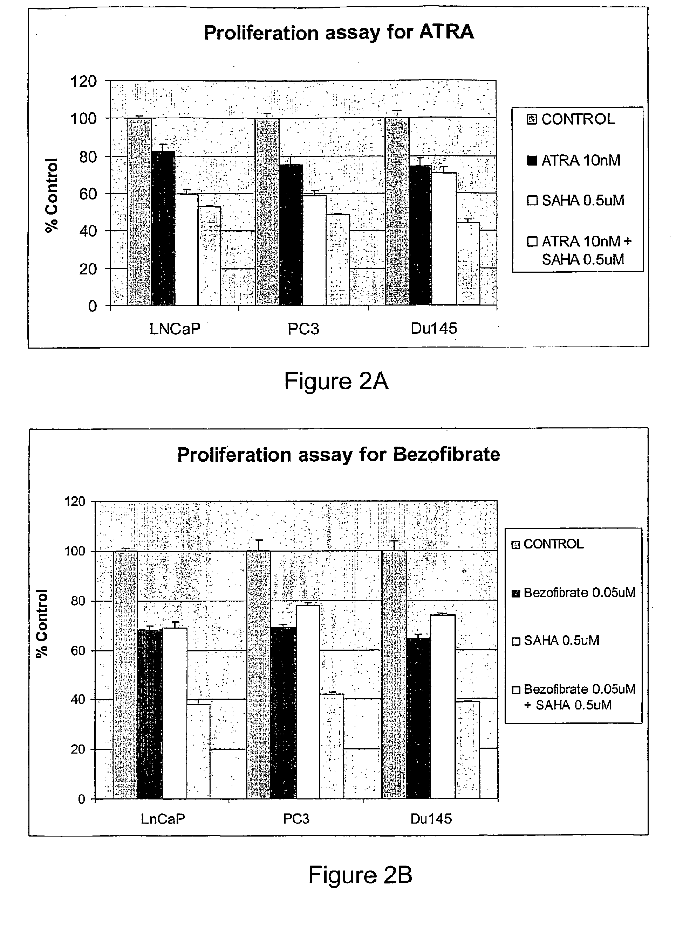

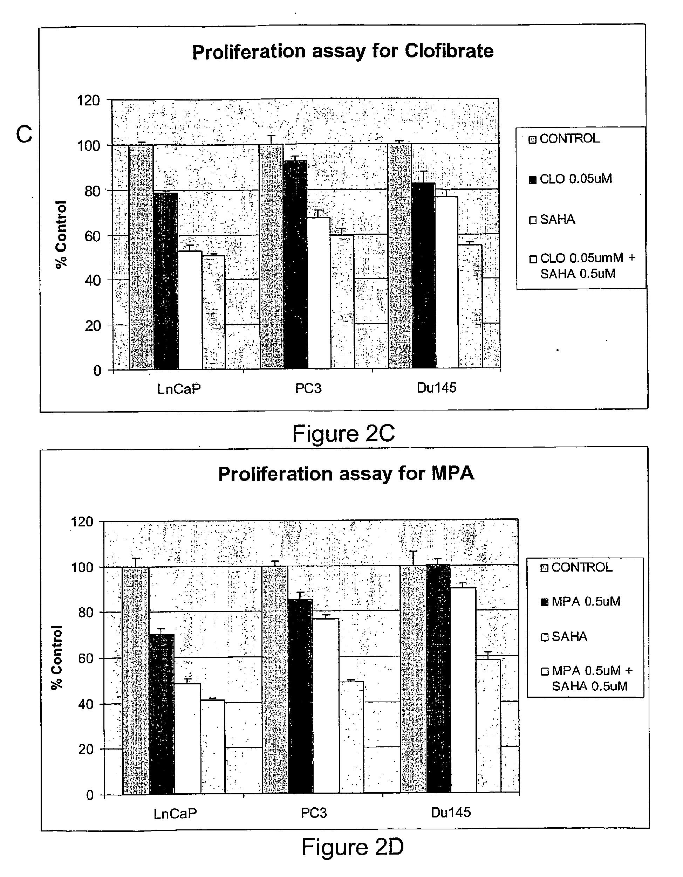

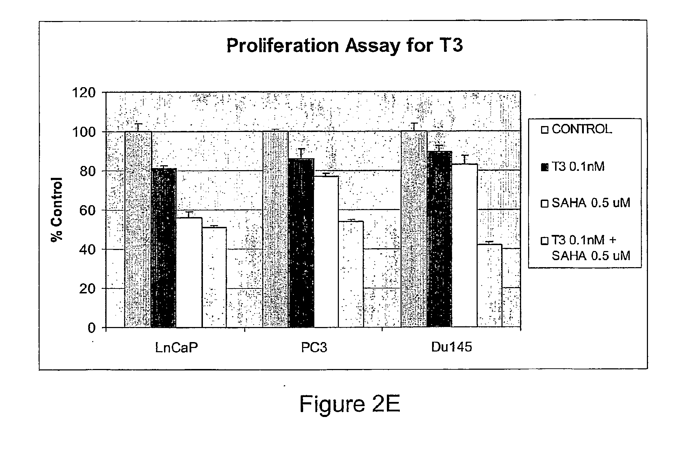

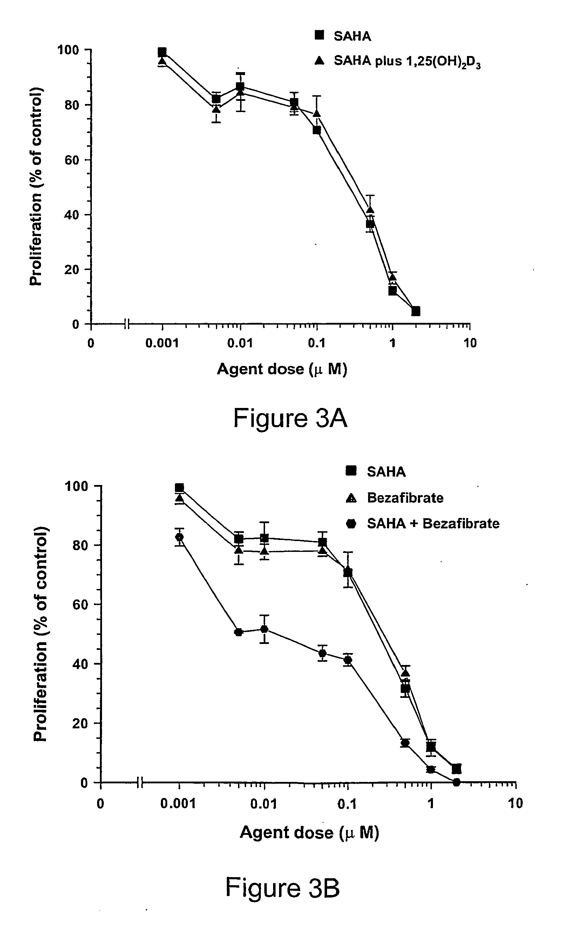

Major issues concerning therapy with HDAC inhibitors such as SAHA, are the possible toxicity of these compounds, as they target such fundamental processes, and the short half-life *in vivo*. To improve efficacy the potential for combinatorial activity was investigated by undertaking a series of studies with SAHA in combination with a broad panel of NR ligands. Thus proliferation responses were screened using a dose of SAHA that results in approximately 25% inhibition of proliferation (0.5 µM) in combination with various NR ligands, at doses that can be achieved *in vivo* and therefore can be readily applied to a clinical trial setting. These included all *trans* retinoic acid (ATRA) which binds to RAR/RXR, Chlofibric acid (CA) and bezafibrate (BF) (PPARα and γ), medroxy progesterone acetate (MPA) (ER/PR), Thyroid T₃ (TR) and 1α,25(OH)₂D₃. The response of cells to single and co-treatment was screened in a 96-well plate proliferation assay. Strikingly many of these combinations displayed a range of additive and even synergistic interactions (Figure 2A-E). Notably the interactions of SAHA did not appear to extend to 1α,25(OH)₂D₃ (Figures 3A and 3B). Together these data suggest that the antiproliferative and pro-apoptotic action of a diverse range of NR ligands is suppressed by inappropriate HDAC activity associated with de-regulated co-repressor activity. Furthermore it suggests that the increased expression or activity of NR co-repressors, with associated HDAC activity may globally target a broad panel of NR.

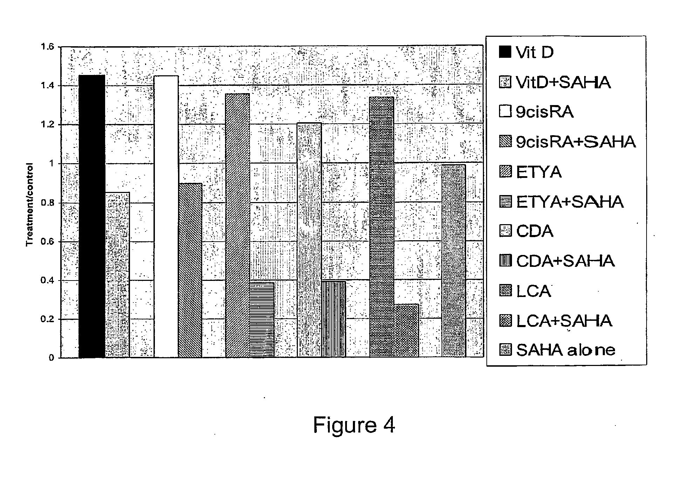

Therefore the capacity to enhance ligand action in bladder cancer models was examined by combination treatment with a clinically relevant histone deacetylase inhibitor, Suberoylanide hydroxamic acid (SAHA). Supportively the cells with the least sensitivity towards ligand displayed additive and synergistic interactions in combination with SAHA, particularly with PPAR, LXR and FXR ligands. Thus the significant ligand responses of RT-4 cells were only modestly influenced by co-treatment with SAHA. By contrast SAHA restored the actions in EJ-28 cells to lithocholic acid (VDR and FXR) and 5,8,11,14-eicosatetraenoic acid (PPARγ) and chenodeoxycholic acid (FXR) (Figure 4). These studies have demonstrated a broad inverse relationship between the sensitivity towards ligand alone and the capacity for co-operative interactions with SAHA co-treatments and support the targeting of dietary sensing nuclear receptors as chemoprevention/chemotherapy targets.

These data are consistent with a model of promoter-specific epigenetic silencing of NR target genes. Targeting epigenetically-repressed PPAR target genes has clinical relevance as a novel therapy for androgen-independent prostate cancer.

### SAHA, but not TSA augments the actions of clinically relevant PPAR and FXR ligands

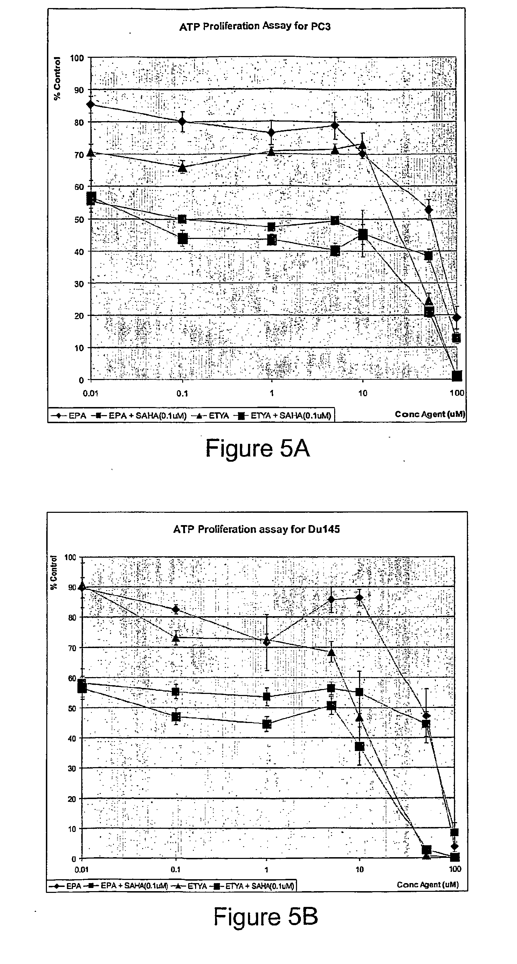

The actions of SAHA and TSA are different with some areas of commonality. Both agents are potent inhibitors of proliferation, and both display a range of combinatorial activities. However these are not the same. TSA augments the actions of 1α,25(OH)₂D₃ (data not shown) whereas SAHA does not (Figure 3A). By contrast SAHA has a profound and strong interaction with bezafibrate (Figure 3B), equally TSA does not augment the actions of bezafibrate, whereas NaB, a broad spectrum HDAC inhibitor does co-operate with bezafibrate, to a more modest extent (data not shown). The interactions of SAHA with the PPARα and PPARγ were examined further using the PPARα and PPARγ specific ligands EPA and ETYA. Interestingly each of these demonstrated the same strong interactions as the pan-agonistic bezafibrate (Figure 5A and 5B).

### SAHA plus bezafibrate induces cell cycle, autophagy responses accompanied by unique gene modulation

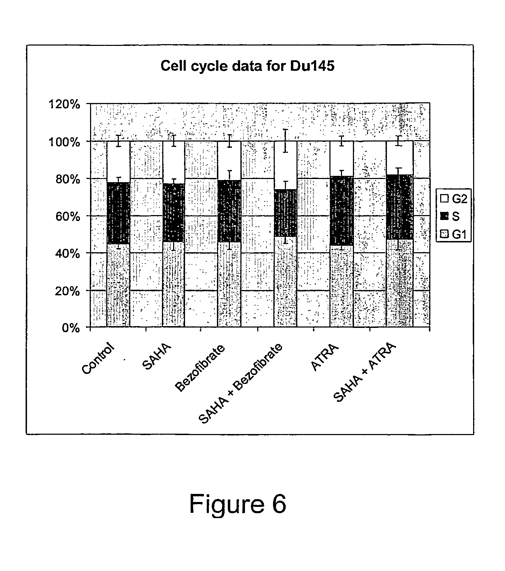

The action of SAHA (0. 5 µM) plus bezafibrate (0.5 µM) on the cell cycle profiles (at 24 and 72 hr), induction of apoptosis (72hr) and the modulation of the known antiproliferative target genes p21^{*(waf1*/*cip1)*} and GADD45α (3 and 8 hr) were examined. The studies showed that the combinatorial changes on the cell cycle profile were generally small, but in the case of the androgen-independent DU 145 the combination of SAHA plus bezafibrate was significantly different than either agent alone (*p*< 0.05) (Figure 6). Parallel studies were undertaken by examining the induction of apoptosis by examining mitochondrial membrane integrity. These studies revealed a profound loss of mitochondrial membrane integrity, without the indication of DNA fragmentation which is characteristic of apoptosis, but rather suggests a role for autophagy programmed cell death. Finally, gene induction with SAHA (0.1 µM) plus bezafibrate (0.1 µM) was screened and it was found that the combination of agents very strongly and significantly induced GADD45α after 8 hr 4.3 fold, whereas each individual agent alone was not significant.

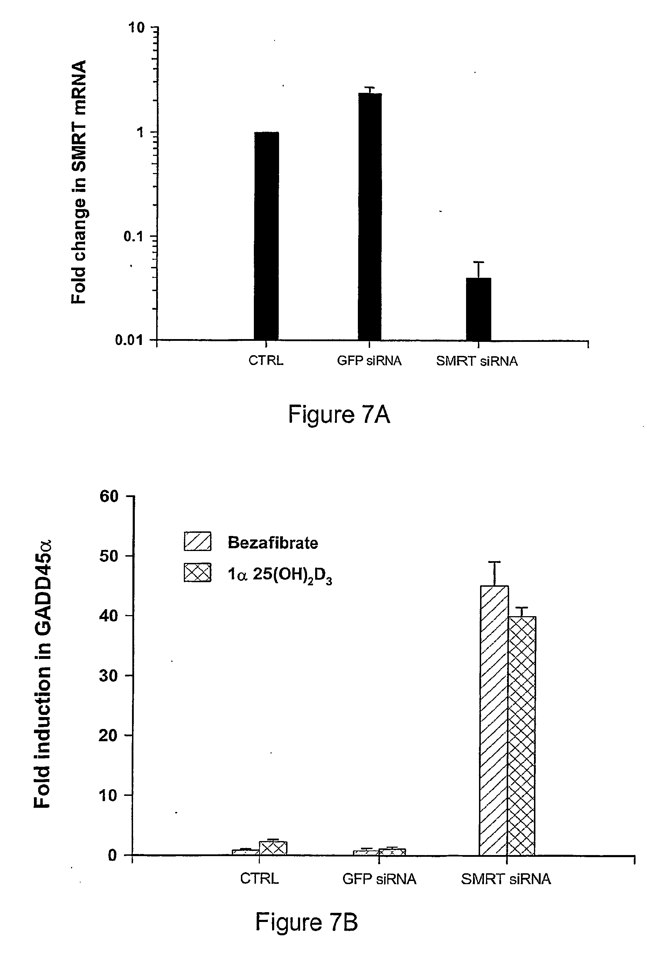

To confirm the role that SMRT plays in suppressing the induction of genes by 1α,25(OH)₂D₃ or bezafibrate SMRT levels in PC-3 cells were knocked down using a small interfering RNA (RNAi) approach (Gene therapy systems, http://www.genetherapysystems.com/) which resulted in a 95% reduction in the basal levels of SMRT mRNA after 72 hr (Figure 7A). It was found that GADD45α induction by either 1α,25(OH)₂D₃ or bezafibrate alone became very strongly enhanced (Figure 7B).

Originally the VDR was described for its central endocrine role in maintenance of serum calcium levels. Similarly the FXR and LXRs were described for their roles in regulating cholesterol and fatty and bile acids metabolism in the enterohepatic system. The expression of these receptors in non-classical tissues, such as the prostate, suggests a broader role in the sensing of dietary lipid molecules. The response to fatty and bile acids is shared by other NRs such as RXRs and the VDR, which can also respond to the secondary bile acid (LCA), to induce the cytochrome P450, CYP3A4. Examination of the VDR, RARs, PPARs, FXR and LXRs reveals that they have in common target genes that regulate the cell cycle (e. g. p21^{*(waf1*/*cip1)*} and GADD45α), differentiation (e. g. NKX3.1 and E-Cadherin) and xenobiotic clearance via cytochrome P450s (e. g. CYP3A4)

Collectively such post-genomic analyses indicate the expression of a wider compliment of nutrient-sensing NRs in for example in normal prostate epithelial cells than hitherto suspected. Thus the prostate can respond to a wide range of dietary lipids and NRs provide a strong molecular mechanism link between nutritional signals, gene regulation and tissue maintainenance. Supportively epidemiological and chemoprevention studies indicate that initiation or progression of prostate cancer may relate to altered intake of micro and macro nutrients (e. g. the balance of omega 3 and 6 fatty acids) and cellular resistance to the receptors which sense these substances. The balance of nutrient ligands is altered by Western-style high fat diets where either key ligands are deficient or NR-detoxification pathways overwhelmed, for example by increased levels of potentially toxic secondary bile acids such as LCA.

The loss of sensitivity to many of these ligands appears clear in malignancy. For example, cancer cell lines from the tissues examined here display a spectrum of sensitivities including complete insensitivity to 1α,25(OH)₂D₃. There is similar, though less complete data to suggest that the same shift towards loss of responsiveness to a range of NR ligands such as those for RAR and PPAR. Prior to work undertaken by the inventors, the mechanisms at the basis of this apparent hormonal insensitivity were unclear and their resolution will facilitate the greater clinical application of NR ligands as anticancer/antiproliferative agents.

Mechanisms that maintain the boundaries between heterochromatin and euchromatin are essential for correct cell-specific gene transactivation. Ligand activation of NR and recruitment of co-activator complexes initiates local chromatin remodeling and thus the dynamic, ligand-regulated, balance between co-activators and co-repressors plays an important role in determining these boundaries. Reflective of this role, alterations in NR co-repressor and co-activator expression have been described in numerous cancers, for example androgen and estrogen receptor co-activators inappropriately enhance the transcriptional activity of androgens and estrogens in breast and prostate cancer respectively. As indicated earlier, a clear example of this is the PML-RARα-fusion proteins in acute promyelocytic leukaemia, driving insensitivity towards all *trans* retinoic acid. Although not wishing to be limited by any theory, the inventors hypothesise that the activity of NR co-repressors is enhanced, resulting in epigenetic suppression of the abilities of a range of ligands to transactivate antiproliferative target genes.

In support of this model the inventors have now shown that cell lines, malignant primary cultures and tumour biopsies have significantly elevated NCoR2/SMRT , NCoR1 levels whilst TRIP15/Alien is seldom altered. Using PC-3 prostate cancer and EJ-28 bladder cancer cells as models with reduced nuclear receptor ligand sensitivity and elevated co-repressor levels, it has been demonstrated that the antiproliferative effects of various ligands can be significantly enhanced by co-treatment with low doses of SAHA.

Together these data underscore the concept that inappropriate, HDAC activity is suppressing the activity of promoters for antiproliferative target genes. Thus bezafibrate/SAHA combinations enhanced the induction of GADD45α, which may be a common target for NR action, repressed by a common epigenetic mechanism. Equally importantly siRNA strategies to NCoR2/SMRT relieved this repression and allowed GADD45α to become very strongly induced by bezafibrate

Taken together, these findings support a model whereby elevated NCoR2/SMRT increases the prevalence of NCoR2/SMRT-HDAC3 repressive complexes, which selectively sustain local histone deacetylation in the promoter/enhancer regions of key antiproliferative target genes.

Current therapeutic strategies involve a combination of radiotherapy and radical prostatectomy, and eventually androgen ablation. These therapies are aggressive, with many side-effects and ultimately lead to predominance of androgen-independent tumours. HDAC inhibitors such as butyrate derivatives, TSA and more recently SAHA are being investigated for a potential role in chemotherapy. Major issues concerning therapy with HDAC inhibitors are the possible toxicity of these compounds, as they target such fundamental processes, and the short half-life *in vivo*. Similarly it is unclear which class of HDAC enzymes is critical to be targeted. SAHA is one of the more promising HDAC compounds and therefore the combination of SAHA with readily tolerated ligands represents an attractive, more focused and sustained 'anticancer' regime, representing a new avenue in the treatment of prostate, bladder cancer and breast cancer, irrespective of conventional diagnostic indicators such as established sex steroid receptors.

## Claims

1. The use of a therapeutically effective amount of a nuclear receptor ligand and an HDAC inhibitor in the manufacture of a medicament or respective medicaments for the treatment of cancer, **characterised in that** said nuclear receptor ligand is a PPAR ligand selected from bezafibrate, eicosapentanoic acid (EPA) and 5, 8, 11, 14-eicosatetraenoic acid (ETYA) or an FXR ligand selected from chenodeoxycholic acid (CDA) and lithocholic acid (LCA), and said HDAC inhibitor is suberoylanilide hydroxamic acid (SAHA).

2. A synergistic combination of an HDAC inhibitor and a nuclear receptor ligand for use in reducing proliferation of or inducing programmed cell death in neoplastic cells, wherein the HDAC inhibitor is suberoylanilide hydroxamic acid (SAHA), and the nuclear receptor ligand is a PPAR ligand selected from bezafibrate, eicosapentanoic acid (EPA) and 5, 8, 11, 14-eicosatetraenoic acid (ETYA), or an FXR ligand selected from chenodeoxycholic acid (CDA) and lithocholic acid (LCA).

3. The use of a nuclear receptor ligand as a synergist in combination with an HDAC inhibitor in the manufacture of a medicament or respective medicaments for the treatment of cancer wherein said nuclear receptor ligand is a PPAR ligand selected from bezafibrate, eicosapentanoic acid (EPA) and 5, 8, 11, 14-eicosatetraenoic acid (ETYA) or an FXR ligand selected from chenodeoxycholic acid (CDA) and lithocholic acid (LCA), and said HDAC inhibitor is suberoylanilide hydroxamic acid (SAHA).

4. The use according to Claim 3 wherein said nuclear receptor ligand is a ligand for PPARα or PPARγ.

5. The use of a nuclear receptor ligand and an HDAC inhibitor in the manufacture of a medicament or respective medicaments for the reduction or prevention of proliferation of neoplastic cells, **characterised in that** said nuclear receptor ligand is a PPAR ligand selected from bezafibrate, eicosapentanoic acid (EPA) and 5, 8, 11, 14-eicosatetraenoic acid (ETYA) or an FXR ligand selected from chenodeoxycholic acid (CDA) and lithocholic acid (LCA), and said HDAC inhibitor is suberoylanilide hydroxamic acid (SAHA).

6. The use of a nuclear receptor ligand and an HDAC inhibitor in the manufacture of a medicament or respective medicaments for the induction of programmed cell death in neoplastic cells, **characterised in that** said nuclear receptor ligand is a PPAR ligand selected from bezafibrate, eicosapentanoic acid (EPA) and 5, 8, 11, 14-eicosatetraenoic acid (ETYA) or an FXR ligand selected from chenodeoxycholic acid (CDA) and lithocholic acid (LCA) and said HDAC inhibitor is suberoylanilide hydroxamic acid (SAHA).

7. An in vitro method of reducing proliferation and/or inducing programmed cell death of neoplastic cells exhibiting abnormal expression or activity of a co-repressor protein, comprising contacting said cells with an HDAC inhibitor and an anti-proliferative and/or programmed cell death-inducing gene trans-activating factor, whereby to induce expression of said anti-proliferative and/or programmed-cell death-inducing gene, **characterised in that** said gene tran-activating factor is a PPAR ligand selected from bezafibrate, eicosapentanoic acid (EPA) and 5, 8, 11, 14-eicosatetraenoic acid (ETYA) or an FXR ligand selected from chenodeoxycholic acid (CDA) and lithocholic acid (LCA), and said HDAC inhibitor is suberoylanilide hydroxamic acid (SAHA).

8. The method according to Claim 7 wherein the abnormally expressed co-repressor protein is selected from NCoR2/SMRT, NCoR1 and TRIP15/Alien.

## Patentansprüche

1. Verwendung einer therapeutisch wirksamen Menge eines nukleären Rezeptorliganden und eines HDAC-Hemmers bei der Herstellung eines Medikaments oder entsprechender Medikamente für die Behandlung von Krebs, **dadurch gekennzeichnet, daß** der nukleäre Rezeptorligand ein PPAR-Ligand, ausgewählt aus Bezafibrat, Eicosapentaensäure (EPA) und 5,8,11,14-Eicosatetraensäure (ETYA), oder ein FXR-Ligand, ausgewählt aus Chenodesoxycholsäure (CDA) und Lithocholsäure (LCA), ist und der HDAC-Hemmer Suberoylanilidhydroxamsäure (SAHA) ist.

2. Synergistische Kombination eines HDAC-Hemmers und eines nukleären Rezeptorliganden zur Verwendung beim Verringern der Proliferation von oder Induzieren von programmiertem Zelltod in neoplastischen Zellen, wobei der HDAC-Hemmer Suberoylanilidhydroxamsäure (SAHA) ist und der nukleäre Rezeptorligand ein PPAR-Ligand, ausgewählt aus Bezafibrat, Eicosapentaensäure (EPA) und 5,8,11,14-Eicosatetraensäure (ETYA), oder ein FXR-Ligand, ausgewählt aus Chenodesoxycholsäure (CDA) und Lithocholsäure (LCA), ist.

3. Verwendung eines nukleären Rezeptorliganden als Synergist in Kombination mit einem HDAC-Hemmer bei der Herstellung eines Medikaments oder entsprechender Medikamente für die Behandlung von Krebs, wobei der nukleäre Rezeptorligand ein PPAR-Ligand, ausgewählt aus Bezafibrat, Eicosapentaensäure (EPA) und 5,8,11,14-Eicosatetraensäure (ETYA), oder ein FXR-Ligand, ausgewählt aus Chenodesoxycholsäure (CDA) und Lithocholsäure (LCA), ist und der HDAC-Hemmer Suberoylanilidhydroxamsäure (SAHA) ist.

4. Verwendung nach Anspruch 3, wobei der nukleäre Rezeptorligand ein Ligand für PPARα oder PPARγ ist.

5. Verwendung eines nukleären Rezeptorliganden und eines HDAC-Hemmers bei der Herstellung eines Medikaments oder entsprechender Medikamente für die Verringerung oder Verhinderung der Proliferation von neoplastischen Zellen, **dadurch gekennzeichnet, daß** der nukleäre Rezeptorligand ein PPAR-Ligand, ausgewählt aus Bezafibrat, Eicosapentaensäure (EPA) und 5,8,11,14-Eicosatetraensäure (ETYA), oder ein FXR-Ligand, ausgewählt aus Chenodesoxycholsäure (CDA) und Lithocholsäure (LCA), ist und der HDAC-Hemmer Suberoylanilidhydroxamsäure (SAHA) ist.

6. Verwendung eines nukleären Rezeptorliganden und eines HDAC-Hemmers bei der Herstellung eines Medikaments oder entsprechender Medikamente für die Induktion von programmiertem Zelltod in neoplastischen Zellen, **dadurch gekennzeichnet, daß** der nukleäre Rezeptorligand ein PPAR-Ligand, ausgewählt aus Bezafibrat, Eicosapentaensäure (EPA) und 5,8,11,14-Eicosatetraensäure (ETYA), oder ein FXR-Ligand, ausgewählt aus Chenodesoxycholsäure (CDA) und Lithocholsäure (LCA), ist und der HDAC-Hemmer Suberoylanilidhydroxamsäure (SAHA) ist.

7. In-vitro-Verfahren zum Verringern von Proliferation und/oder Induzieren von programmiertem Zelltod von neoplastischen Zellen, die abnormale Expression oder Aktivität von einem Corepressor-Protein zeigen, umfassend Inkontaktbringen der Zellen mit einem HDAC-Hemmer und einem anti-proliferativen und/oder programmierten Zelltod induzierenden Gen-trans-aktivierenden Faktor, um dadurch Expression des anti-proliferativen und/oder programmierten Zelltod induzierenden Gens zu induzieren, **dadurch gekennzeichnet, daß** der Gen-trans-aktivierende Faktor ein PPAR-Ligand, ausgewählt aus Bezafibrat, Eicosapentaensäure (EPA) und 5,8,11,14-Eicosatetraensäure (ETYA), oder ein FXR-Ligand, ausgewählt aus Chenodesoxycholsäure (CDA) und Lithocholsäure (LCA), ist und der HDAC-Hemmer Suberoylanilidhydroxamsäure (SAHA) ist.

8. Verfahren nach Anspruch 7, wobei das abnormal exprimierte Corepressor-Protein aus NCoR2/SMRT, NCoR1 und TRIP15/Alien ausgewählt ist.

## Revendications

1. Utilisation d'une quantité thérapeutiquement efficace d'un ligand de récepteur nucléaire et d'un inhibiteur de HDAC dans la fabrication d'un médicament ou de médicaments respectifs pour le traitement du cancer, **caractérisée en ce que** ledit ligand de récepteur nucléaire est un ligand de PPAR choisi parmi le bézafibrate, l'acide éicosapentanoïque (EPA), et l'acide 5,8,11,14-éicosatétraénoïque (ETYA), ou un ligand de FXR choisi parmi l'acide chénodésoxycholique (CDA) et l'acide lithocholique (LCA), et ledit inhibiteur de HDAC est l'acide subéroylanilide hydroxamique (SAHA).

2. Combinaison synergique d'un inhibiteur de HDAC et d'un ligand de récepteur nucléaire destinée à être utilisée pour la réduction de la prolifération de cellules néoplasiques ou pour l'induction d'une mort cellulaire programmée dans des cellules néoplasiques, l'inhibiteur de HDAC étant l'acide suberoylanilide hydroxamique (SAHA), et le ligand de récepteur nucléaire étant un ligand de PPAR choisi parmi le bézafibrate, l'acide éicosapentanoïque (EPA), et l'acide 5,8,11,14-éicosatétraénoïque (ETYA), ou un ligand de FXR choisi parmi l'acide chénodésoxycholique (CDA) et l'acide lithocholique (LCA).

3. Utilisation d'un ligand de récepteur nucléaire en tant que synergiste en combinaison avec un inhibiteur de HDAC dans la fabrication d'un médicament ou de médicaments respectifs pour le traitement du cancer, ledit ligand de récepteur nucléaire étant un ligand de PPAR choisi parmi le bézafibrate, l'acide éicosapentanoïque (EPA), et l'acide 5,8,11,14-éicosatétraénoïque (ETYA), ou un ligand de FXR choisi parmi l'acide chénodésoxycholique (CDA) et l'acide lithocholique (LCA), et ledit inhibiteur de HDAC étant l'acide subéroylanilide hydroxamique (SAHA).

4. Utilisation selon la revendication 3 dans laquelle ledit ligand de récepteur nucléaire est un ligand de PPARα ou de PPARγ.

5. Utilisation d'un ligand de récepteur nucléaire et d'un inhibiteur de HDAC dans la fabrication d'un médicament ou de médicaments respectifs pour la réduction ou la prévention de la prolifération de cellules néoplasiques, **caractérisée en ce que** ledit ligand de récepteur nucléaire est un ligand de PPAR choisi parmi le bézafibrate, l'acide éicosapentanoïque (EPA), et l'acide 5,8,11,14-éicosatétraénoïque (ETYA), ou un ligand de FXR choisi parmi l'acide chénodésoxycholique (CDA) et l'acide lithocholique (LCA), et ledit inhibiteur de HDAC est l'acide subéroylanilide hydroxamique (SAHA).

6. Utilisation d'un ligand de récepteur nucléaire et d'un inhibiteur de HDAC dans la fabrication d'un médicament ou de médicaments respectifs pour l'induction d'une mort cellulaire programmée dans des cellules néoplasiques, **caractérisée en ce que** ledit ligand de récepteur nucléaire est un ligand de PPAR choisi parmi le bézafibrate, l'acide éicosapentanoïque (EPA), et l'acide 5,8,11,14-éicosatétraénoïque (ETYA), ou un ligand de FXR choisi parmi l'acide chénodésoxycholique (CDA) et l'acide lithocholique (LCA), et ledit inhibiteur de HDAC est l'acide subéroylanilide hydroxamique (SAHA).

7. Méthode in vitro pour la réduction de la prolifération de cellules néoplasiques et/ou pour l'induction d'une mort cellulaire programmée dans des cellules néoplasiques, lesdites cellules néoplasiques présentant une activité ou une expression anormale d'une protéine co-répresseur, consistant à mettre en contact lesdites cellules avec un inhibiteur de HDAC et un facteur de transactivation de gène anti-prolifératif et/ou de gène induisant une mort cellulaire programmée, de manière à induire l'expression dudit gène anti-prolifératif et/ou gène induisant une mort cellulaire programmée, **caractérisée en ce que** ledit facteur de transactivation de gène est un ligand de PPAR choisi parmi le bézafibrate, l'acide éicosapentanoïque (EPA), et l'acide 5,8,11,14-éicosatétraénoïque (ETYA), ou un ligand de FXR choisi parmi l'acide chénodésoxycholique (CDA) et l'acide lithocholique (LCA), et ledit inhibiteur de HDAC est l'acide subéroylanilide hydroxamique (SAHA).

8. Méthode selon la revendication 7 dans laquelle la protéine co-répresseur anormalement exprimée est choisie parmi NCoR2/SMRT, NCoR1 et TRIP15/Alien.
